# EUROPEAN PATENT APPLICATION

(11) **EP 2 229 824 A1**
(43) Date of publication of application: **22.09.2010**
(21) Application number: 09154996.4
(22) Date of filing: 12.03.2009
(51) Int. Cl.: A23L 1/30, A23L 1/305, A23J 1/20, A23L 3/3535, A61K 31/26, A61P 9/10, A61P 35/00

(54) **Covalent milk protein/isothiocyanate complexes**

(71) Applicant: Nestec S.A., 1800 Vevey (CH)
(72) Inventor: Rade-Kukic, Koraljka, 1018 LAUSANNE (CH); Schmitt, Christophe, 1077 SERVION (CH)
(74) Representative: Marquardt, Ulf

(57) **Abstract**

The present invention relates to the field of complexes. Embodiments of the present invention relate to food-grade covalent complexes containing at least one milk protein and at least one ITC-compound and to the uses of such complexes, e.g., to reduce the perceived pungency of ITC-compounds, to produce antimicrobial effects and/or to form and stabilize emulsions and/or foams.

## Description

The present invention relates to the field of complexes.
Embodiments of the present invention relate to food-grade covalent complexes containing at least one milk protein and at least one ITC-compound and to the uses of such complexes, e.g., to reduce the perceived pungency of ITC-compounds, to produce antimicrobial effects and/or to form and stabilize emulsions and/or foams.

Glucosinolates (GLS) are natural sulfur-containing phytochemicals synthesized in vegetables from the Brassicaceae family such as broccoli, kale, cabbage, cauliflower, Brussels sprouts, turnip or mustard greens. Consumption of these vegetables has been associated with reduced incidence of cancer, particularly of lung, stomach, colon, and rectal cancer. Their protective effect is largely attributed to the presence of GLS that possess a common structure comprising a β-D-thioglucose group, a sulfonated moiety, and a variable side chain derived from methionine, tryptophan, phenylalanine or various branched chain amino acids.

GLS are chemically stable and are considered to be biologically inactive, but when brought into contact with the plant enzyme myrosinase, or human colonic bacteria, they break down releasing glucose and an unstable aglycon. The latter undergoes spontaneous rearrangement into, depending on hydrolysis conditions, different possible products including nitriles, thiocyanates, epithionitriles or isothiocyanates (ITC).

ITC are considered to be amongst the most potent naturally occurring bioactive compounds intervening in the process of cancer development. They can affect the endogenous antioxidant potential, enhance detoxification mechanisms and induce apoptosis in undifferentiated cells. ITC have also been described as efficient antibacterial compounds.

US20030235634A1 describes a method for extracting nonpolar isothiocyanates from plants and dietary supplements containing same.

US200330064131 describes a preparation of isothiocyanate product from cruciferous plant material prepared by maintaining the plant material at specified temperature and subjecting isothiocyanate product to further processes.

GB2404659A is a fractionation of mustard seeds involving combining cracked mustard seed stock with water to create activated mustard slurry, converting glucosinolate sinigrin into fraction allyl oil, and separating this fraction from the remainder of mustard slurry.

WO0215722 describes a method for treating Helicobacter infections comprising administration of a composition comprising a glucosinolate or an isothiocyanate or a derivative.

DE2045408 describes a process of addition of thiocyanate or isothiocyanate in milk that is not acidified properly for the production of yoghurt or other types of fermented milk products.

W02005032283A1 is about a composition for preserving solid food products comprising a moisture-sensitive isothiocyanate compound and a hygroscopic carrier, wherein the composition is substancially free of sorbic acid, benzoic acid, and salts thereof.

W02006133789A1 describes consumer products comprising a natural preservative system. The preservative system has a mixture of aliphatic and aromatic isothiocyanates in a ratio of 1:2 to 1:25 and is suitable for use in variety of consumer products.

In a recent review, Zhang (Cancer-preventive isothiocyanates: measurements of human exposure and mechanism of action. 2004. Mutation Res, 555, 173-190) reported on the results of epidemiological studies showing inverse correlation between the risk of developing lung, breast or colon cancer and the dietary consumption of ITC's.

US20060258599A1 describes the use of isothiocyanates for treating a subject having cystic fibrosis.

W02006118941A1 reports on the suppression of ultraviolet light-induced skin carcinogenesis in a subject involving administration of sulforaphane or its analogue, isothiocyanate or glucosinolate.

However, while ITC have a high potential as bioactive molecules, they have a flavour that is generally perceived as unpleasant. For example they influence the flavor of *Brassica* vegetables. Of course, they also influence the taste of food products negatively, to which they are added to.

Drewnowski and Gomez-Carneros (Bitter taste, phytonutrients, and the consumer: a review. 2000. Am J Clin Nutr, 72, 1424-1435) reported that allyl-isothiocyanate occurring in Brassicaceae (especially cabbage, mustard and horseradish) was reported as pungent and lachrymatory compound in sensory experiments.

Hence, while it is generally well known that ITC-compounds have very beneficial properties for health, they are still not sufficiently consumed, because their taste is usually disliked.

There is hence a need in the art to have available a composition comprising ITC-compounds which is pleasant to consume.

It was hence the object of the present invention to improve the state of the art and to provide a composition containing ITC-compounds, which has an acceptable taste.

The inventors were surprised to see that they could achieve this object by the subject matter of the independent claims. The dependant claims further develop the idea of the present invention.

The subject matter of the present invention provides a composition containing ITC, which has no pungent and/or no lachrymatory taste or which has a taste that is perceived as less pungent and/or less lachrymatory than state of the art compositions comprising ITC-compounds.

The present inventors provide a composition comprising a covalent complex of at least one isothiocyanate compound and at least one milk protein.

The inventors have also discovered that these complexes exhibit excellent emulsifying and foaming properties. This further enhances the applicability of the complexes in the food industry.

β-Lactoglobulin (BLG), the major bovine whey protein is known to contain 1 free sulfhydryl group and 16 amino groups. It is hence able to form covalent complexes with ITC.

In the present invention, the inventors show that a complex formation between BLG, as one example of a milk protein, and at least one ITC-compound enables reducing the undesirable taste of the ITC-compound. This allows it to add ITC-compounds to many food products, which so far could not be enriched with ITC-compounds due to taste reasons. The present invention allows it now to deliver ITC-compounds through various food products. In addition, the formation of covalent complexes enables modifying the protein's functional properties, allowing their wider and/or improved usage as food ingredients.

Hence, one embodiment of the present invention is a composition comprising a covalent complex. The covalent complex comprises at least one ITC-compound and at least one milk protein.

By the term "complex" is meant any form of association between at least one ITC-compound and at least one milk protein.

The complexes of the invention are covalently bound complexes. The covalent interaction between ITC-compounds and milk proteins is presently thought to occur upon a nucleophile addition of the isothyocynate, e.g., at elevated pH values either to free sulfhydryl or α- or ε-amino groups of the protein (Rawel, Kroll and Schröder. In vitro enzymatic digestion of benzyl- and phenylisothiocyanate-derivatized food proteins. 1998. J Agric Food Chem, 46, 5103-5109).

The term "milk protein" comprises any protein occurring in milk, as well as any milk or milk derived protein fraction.

The milk may be for example bovine milk, sheep milk, goat milk, horse milk, camel milk or soy milk. Bovine milk is preferred.

In an alternative preferred embodiment of the present invention is the milk protein a whey protein, e.g., sweet whey protein or acid whey protein, preferably from bovine origin.

Hence, the milk protein may be bovine milk or a bovine milk derived protein fraction. For example the milk protein may be selected from the group consisting of the protein fraction of skimmed milk, milk protein concentrate, milk protein isolate, micellar casein, caseinate, αₛ₁-casein, αₛ₂-casein, β-casein, κ-casein, the protein fraction of acid or sweet whey, whey protein concentrate, whey protein isolate, α-lactalbumin, β-lactoglobulin, bovine serum albumin, lactoferrin, or combinations thereof.

ITC-compounds are all compounds including the isothiocyanate group. Isothiocyanate may be formed by substituting sulfur for oxygen in the isocyanate group. ITC-compounds include organic isothiocyanates with the general formula R-N=C=S. R may be selected from the group consisting of C₁-C₆ alkyl groups, C₁-C₆ alkenyl groups, C₁-C₆ alkinyl groups, C₅-C₁₈ aromatic groups, all optionally containing 1-6 heteroatoms selected from the group consisting of O, N, P, S, and combinations thereof.

Typical isothiocyanates that may be used in the framework of the present invention include organic isothiocyanates such as methyl isothiocyanate, allyl isothiocyanat, phenylethyl isothiocyanate, sulforaphane, phenethyl isothiocyanate, benzyl isothiocyanate, 3-methylthiopropyl isothiocyanate, and combinations thereof.

The stochiometry of the complexes will depend, for example, on the type of protein used, the number and stereochemical availability of ITC binding sites, as well as on the kind and relative amount of the ITC-compound used when preparing the complex. E.g., β-lactoglobulin (BLG), the major bovine whey protein is known to contain 1 free sulfhydryl group and 15-16 amino groups which all may form a covalent bond with an ITC functional group. Importantly and advantageously, e.g., by choosing a particular milk protein and a particular ITC-compound in appropriate relative amounts the nature of the resulting complex can be adjusted and fine tuned to the needs in a final product.

Typically, isothiocyanate and milk protein is present in the complex in a molar ratio in the range of about 100:1 to 1:10, preferably in a molar ratio in the range of about 10:1 to 1:10, most preferable in the range of about 1:1.

The composition of the present invention may have any pH value, but in particular for food products it may preferably have a pH in the range of about 2 to 9, preferably of about 3 to 7.

It was found that the complexes of the present invention may be used to stabilize emulsions.

An emulsion is a mixture of two immiscible liquids. One liquid (the dispersed phase) is dispersed in the other (the continuous phase). For example an emulsion may be an oil-in-water or a water-in-oil emulsion. Many emulsions are oil/water emulsions, with dietary fats being one common type of oil encountered in everyday life. Examples of emulsions include butter, margarine, milk, cream, mayonnaises and/or vinaigrettes. In milk and cream, water surrounds droplets of fat (an oil-in-water emulsion).

It was shown that the complexes of the invention provide a very good emulsifying stability. Consequently, the complexes can be used to stabilize emulsions over time. Additionally, the complexes of the present invention may also be used to stabilize emulsions under heated conditions.

For example, the present inventors were able to show that the complexes of the present invention allowed it to stabilize an emulsion at an acidic pH (e.g., pH 4) and at elevated temperatures (e.g., 85 °C).

The emulsions remained stable for 33 days without any sign of creaming or oiling off.

Consequently, the complexes of the present invention may be used as emulsifiers and/or stabilisers, for example in in food, cosmetic and/or pharmaceutical products.

For example, the composition of the present invention may comprise or consist of a foam. A foam is a substance that is formed by dispersing many gas bubbles in a liquid, a semi solid or a solid continuous phase.

The composition of the present invention may be a food product, a neutraceutical, a food additive, a medicament, a cream for topical application or a drink.

The compositions of the present invention may also be used for the preparation of a product comprising ITC-compounds, for example comprising an emulsion and/or a foam comprising ITC-compounds. This product may then be also a food product, a neutraceutical, a food additive, a medicament, a cream for topical application or a drink.

Compositions and/or products comprising the complexes of the present invention are preferably selected from desserts, frozen desserts, dairy products, petfood, culinary products, clinical nutrition products etc. In particular, they may include sauces, soups, mayonnaises, salad dressings, creams, ice cream, chocolate, mousses, and/or milk.

Typical food products may be also selected from the group consisting of fillings, dips, sauces, mayonnaises, spreads, toppings, dairy-based products, milk and/or cream based foams and/or emulsions, a salad dressings, soups, beverages or oral food supplements.

The complexes or compositions of the invention may also be used in cosmetic products such as creams, foams, mousses, gels, shampoos, emulsions, etc.

Pharmaceutical products to which the complexes of the invention may be added include tablets, capsules, syrups, etc.

The present invention also extends to the complexes and/or compositions of the present invention for the treatment and/or prevention of the disorders mentioned herein.

When used as emulsifier and/or stabiliser, the complexes are preferably present in the product in an amount of 0.01 to 10 wt%, preferably 0.1 - 5 wt%, most preferably 0.1 - 0.5 wt% of said product. It has indeed been found that the emulsifying and stabilising properties are optimal at low concentrations. The products of the invention thus provide the advantage that they are highly effective emulsifiers and/or stabilisers.

The complexes of the present invention may be prepared simply by mixing appropriate amounts of at least one ITC-compound and at least one milk protein in a liquid, preferably water based medium and by allowing the complex to form. Optionally, the pH may be adjusted appropriately and the mixture may be heated, e.g., to 85°C for 15 min and/or agitated.

The resulting complex has the advantage of being food-grade. A material is food-grade if it consists of compounds that are approved for human or animal consumption.

The complexes are highly efficient as an emulsifier and/or stabiliser in compositions to which it is added. Furthermore, the complex comprises only natural ingredients such that it is more appealing than traditional emulsifiers which consist of chemically modified or synthesised products. Also, the complexes of the invention are versatile in terms of the products they may be included into. For instance, by tuning the choice of protein and ITC-compound, they function as emulsifiers and/or stabilisers over a wide pH range. For instance, they can be used in acidic products having a pH of about 4.5 such as mayonnaise as well as in products having a pH over 6.5 such as milk.

Additionally, the solution after complex formation may be subjected to ultrafiltration. This has the advantage of separating the complexes of the invention from free ITC.

In a preferred embodiment, the solution is then dried by any method known in the art such as spray-drying, freeze-drying or vacuum-drying.

The complexes of the invention can therefore be in the form of a solution, a gel, or a dried powder.

In a further embodiment, the protein - ITC-compound complexes may be dried in the presence of further ingredients. Alternatively, the dried protein - ITC-compound complex may be mixed with other dried ingredients.

The resulting products can be for instance a milk powder or dehydrated soup powder comprising the complexes of the invention.

Isothiocyanates are known to be instable, in particular heat instable. Hence, they tend to loose at least a part of their health benefits with food processing and storage times. It would however be desirable to increase the health benefits of Brassicaceae plants or plant products, and isothiocyanate containing products in general, even after food processing and long storage times.

ITC are also volatile, so their quantity decreases over time as well. The complexes of the present invention avoid such a loss.

The present inventors have found that the complexes described in the framework of the present invention allow it to preserve isothiocyanates and their health benefits even after exposure to heat and/or long storage times.

Hence, the composition of the present invention may be used to preserve isothiocyanates, for example during long storage times and or during food processing, in particular involving heat treatments.

The composition of the present invention may also be used to reduce the pungent and/or lachrymatory taste of ITC-compounds. This is of particular importance for food compositions or drinks containing ITC-compounds. Since the complex described in the present invention may be used to provide a foam and/or an emulsion containing ITC-compounds it may also be used to reduce the pungent and/or lachrymatory taste of foams and/or emulsions containing ITC-compounds.

Pungency is also called piquancy. It is a sharp and biting sensory impression. Food that causes this sensation is often called "spicy". Pungency caused by ITC-compounds, for example allyl isothiocyanate; or capsaicin, is currently thought to be caused by activation of the heat thermo- and chemosensitive TRP ion channels including TRPV1 and TRPA1 nociceptors.

A lachrymatory taste is a sensation caused for example by an ITC-compound that stimulates the corneal nerves in the eyes to cause tearing, pain, and possibly even temporary blindness.

The present inventors were furthermore able to show that the composition of the present invention may be used to produce an antimicrobial effect. For example, *Escherichia* coli and *Staphylococcus aureus* dispersions exhibited after incubation with milk protein-ITC-compound covalent complex a reduced optical density (OD).

Consequently, the compositions of the present invention may be used to produce an antimicrobial effect in a product, e.g., a food product. In particular the growth of Escherichia coli and/or Staphylococcus aureus may be inhibited.

Alternatively, the composition of the present invention may also be used for the production of a product to treat or prevent disorders linked to bacterial infection, in particular Escherichia coli and/or Staphylococcus aureus infections.

The product may be a food product, a nutraceutical, a food additive, a drink, a cream for topical administration and/or a pharmaceutical composition, for example.

In a further embodiment the composition of the present invention may be used for the production of a product to inhibit carcinogenesis and/or tumorigenesis, in particular (UV) light-induced skin carcinogenesis. ITC-compounds are presently thought to inhibit carcinogenesis, for example through inhibition of cytochrome P450 enzymes, which produce polar epoxy-diols which can cause mutations and induce cancer development.

In yet a further embodiment the composition of the present invention may be used for the production of a product to treat cystic fibrosis. US20060258599A1 describes the use of isothiocyanates for treating a subject having cystic fibrosis.

In another embodiment the composition of the present invention may be used for the production of a product to treat or prevent inflammatory disorders; in particular inflammatory disorders that can be treated or prevented by the induction of phase 2 enzymes. Inflammatory disorders that can be treated or prevented by the induction of phase 2 enzymes are known to those of skill in the art and are for example described by Juurlink, Therapeutic potential of dietary phase 2 enzyme inducers in ameliorating diseases that have an underlying inflammatory component, Can J. Physiol., Volume 79, 2001, p.266ff.

Two typical examples of inflammatory disorders that may be treated or prevented with the induction of phase 2 enzymes are atherosclerosis and gut inflammation.

Those skilled in the art will understand that they can freely combine all features of the present invention described herein, without departing from the scope of the invention as disclosed. In particular, features described for the uses of the present invention may be applied to the composition and product of the present invention and vice versa.

Further advantages and features of the present invention are apparent from the following Examples and Figures.

Figure 1 shows the binding curves of allyl isothiocyanate (AITC) to β-lactoglobulin (BLG) calculated from the disappearance of -SH or -NH₂ group of the protein after incubation at 25°C and pH 8.5. The fit of the binding curve is plotted as a full line together with the dissociation constant K_{D} and the maximum number of binding sites nₘₐₓ. Vertical bars represent standard deviation.

Figure 2 shows volume stability of foams made with BLG-AITC covalent complexes with or without heat treatment (85°C, 15 min) at pH 4.0 and pH 7.0 for different AITC to protein mixing ratios indicated on the charts.

Figure 3 shows the macroscopic appearance of 10% oil-in-water emulsions made at pH 4.0 and pH 7.0 with 0.25 mM AITC or with BLG-AITC covalent complexes (molar ratio 1:1) with or without heat treatment (85°C, 15 min) after 4 and 26 days of preparation. The emulsions were stored at +4°C.

Figure 4 shows sensory scores related to pungency evaluation of 0.5 mM or 1 mM AITC alone and in the form of a covalent complex with BLG, having an AITC/BLG molar ratio of 1 or 2 (with heat treatment 85°C, 15 min).

Figure 5 shows reduction of optical density (OD) in *Escherichia coli* and *Staphylococcus aureus* dispersions after incubation with BLG-AITC covalent complexes having AITC/BLG molar ratio of 1 to 20. Vertical bars represent standard deviation.

### Examples:

### Example 1: Determination of β-lactoglobulin/allyl isothiocyanate covalent complex formation

β-lactoglobulin (BLG) powder was dispersed in Millipore^{®} water by stirring at room temperature for 1 hour. BLG was purchased from Davisco (Biopure, lot JE 001-3-922). The protein content was 93.5 g/100 g of powder, as determined by Kjeldahl analysis (N x 6.38). The composition in the major whey protein fraction was 89.22% BLG, 6.91% β-lactalbumin, 3.87% bovine serum albumin, as determined by reversed phase HPLC (RP-HPLC); 0.2% fat, 1.5% ash and 4.9% moisture, as specified by the supplier. The mineral composition was as follows: 0.87% Na⁺, <0.004% K⁺, <0.04% Cl⁻, 0.019% Ca²⁺, 0.053% P, 0.002% Mg²⁺, as determined by atomic absorption spectroscopy. Protein solubility at pH 4.6 revealed that 96% of the proteins were in native state.

Concentration of the BLG in the protein solution was 1 mM (1.84% w/w). It was stored over night at 4°C to allow hydration. The next day, it was filtered using a Stericup filtration system (Millipore^{®}) with 0.22 µm GP Express Plus membrane. The pH of filtered solution (pH 7.2) was adjusted to pH 8.5 using 1M NaOH. The amount of 1M NaOH added was about 0.3% of the total volume.

Allyl-isothiocyanate (AITC) solution was prepared freshly prior to the experiment by dissolving AITC in ethanol to obtain a concentration 200 mM (1.98 % v/w). Allyl Isothiocyanate ≥ 98%, was purchased from Sigma (lot 455295).

A volume of 50 mL of mixtures was prepared by mixing 1 mM BLG (pH 8.5) with 200 mM AITC in Millipore^{®} water to obtain AITC to BLG molar ratios from 0.1 to 40. Final concentration of ethanol in all sample solutions was adjusted to 5%. Samples were incubated 24h at room temperature, under agitation (using a roller mixer SRT2, Milian, Switzerland) and protected from light. Upon incubation, contents in free sulfhydryl (-SH) and amino (-NH₂) groups were determined using the Ellman's reagent [5,5'-dithio-bis (2-nitrobenzoic acid); DTNB] (Ellman G L. Tissue sulfhydryl groups. 1959. Arch Biochem Biophys, 82, 70-77) and o-phthaldialdehyde (OPA) methods (Hofmann K, Hamm R. Sulfhydryl and disulfide groups in meats. 1978. Adv Food Res, 24, 1-111), respectively.

To determine the amount of total -SH groups sample mixtures were diluted to the final BLG concentration 50 µM in 200 mM Tris-base (Tris(hydrohymethyl)-aminomethan) buffer containing 1% SDS and 8M urea at pH 8.0. 50 µL of 10 mM DTNB in ethanol was added to 3 mL of these samples and they were left 20 min at room temperature under mild agitation. The absorbance at 412 nm was measured against corresponding reagent blanks (1% ethanol in the buffer with DTNB) using an Uvikon^{®} 810 spectrophotometer (Kontron Instruments, Basel, Switzerland). Samples without DTNB did not absorb at this wavelength. Solutions of AITC in concentrations superior to 0.1 mM, treated under the same conditions as samples, in the presence of DTNB absorbed slightly at the given wavelength. Therefore, absorbance values of samples were corrected for the absorbance of corresponding concentrations of AITC. Concentration of SH groups in sample solutions was assessed according to the previously made standard calibration curve. For that purpose L-Cys (Fluka, Switzerland) dissolved in the same buffer in concentration range 0.005 - 0.1 mM was used.

Quantitative determination of free α-and ε-amino groups was done using the approach based on the method of Goodno et al. (Goodno C. C. Swaisgood H. E., Catignani G. L.; (1981). A fluorimetric assay for available lysine in proteins. Anal.Biochem. 115(1), 203-211.) with modifications done in our laboratory. Briefly, the determination of amino groups was carried out by mixing sample solutions (0.1 mL) with 0.3 % (w/v) N-acetyl-L-cystein (Fluka, Switzerland) in borate buffer pH 9.3 (9.1 mL) and 20 % SDS (w/w; 0.5 mL). After 10 minutes of incubation at 50°C, 3.4 % OPA in methanol (w/v) was added to mixtures (0.3 mL), and they were incubated for another 30 minutes at the same temperature. The final concentration of BLG in 10 mL of sample mixtures was 5 µM. Upon incubation, mixtures were allowed to cool down at room temperature for 30 minutes before absorbance reading was done at 340 nm. The same spectrophotometer as described previously was used. Solutions of AITC in concentrations superior to 0.2 mM, treated under the same conditions as samples, in the presence of OPA absorbed slightly at the given wavelength. Therefore, absorbance values of samples were corrected for the absorbance of corresponding AITC concentrations. Concentration of free (α- and ε-) amino groups in sample solutions was assessed according to the previously made standard calibration curve. For that purpose L-Leu (Fluka, Switzerland), mixed with same solutions and incubated in the same way as described above, in a concentration range 0.002 - 0.15 mM was used.

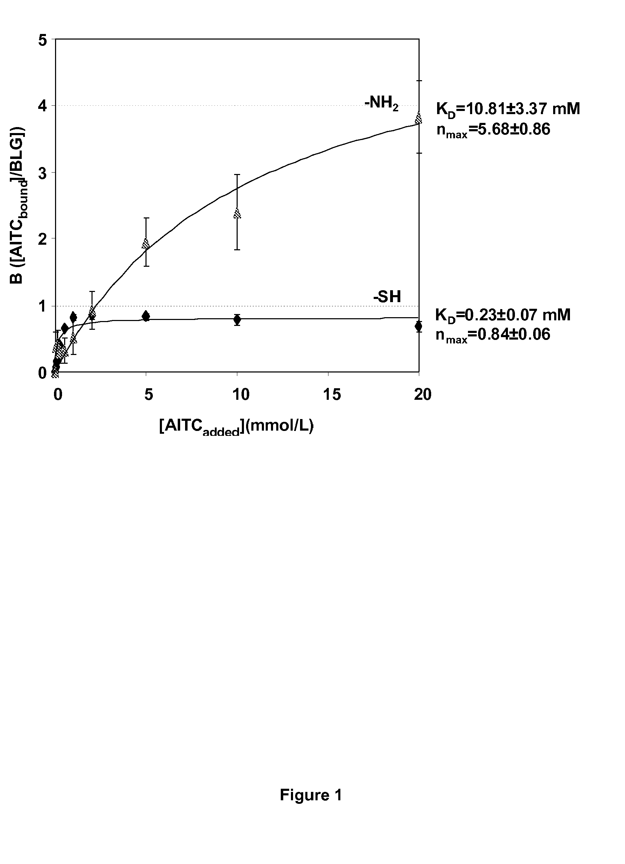

Assuming that one AITC can bind per one -SH group or per one -NH₂ group at the time, the molar binding ratio of AITC to BLG (B_{AITC/BLG}) was estimated from measured values for free -SH and -NH₂ groups. Binding curves were obtained by plotting B_{AITC/BLG} for each reactive group separately against added AITC (fig.1).

The best-fit values for binding parameters (K_{D} and nₘₐₓ) were achieved by applying non-linear least-squares regression using the software Microcal Origin 6.0 (Microcal Software Inc., Northampton, USA). For each binding sites the equation for one site binding was applied.

From figure 1, it can be concluded that AITC was first reacting with the single free sulfhydryl group of BLG, leading to a maximum number of saturation site of 0.84±0.06. This can be explained by the very low value of the calculated dissociation constant K_{D}, leading to a high affinity of ITC for the SH group. Thereafter, ITC was reacting with amino groups which have a lower affinity for ITC (high K_{D}), but a larger number of possible binding sites, owing to the 16 free NH₂ available on the BLG.

### Example 2: Determination of the foaming properties of β-lactoglobulin/allyl isothiocyanate covalent complexes

Foaming properties of samples (prepared as described in example 1) were evaluated using the standardized method proposed by Guillerme C, Loisel W, Bertrand D, Popineau Y. Study of foam stability by video image analysis: Relationship with the quantity of liquid in the foams. 1993. J Text Stud, 24, 287-302. The apparatus used was a standard version of the Foamscan^{™} from Teclis - ITConcept (Longessaigne, France). The principle of the method is to foam a definite quantity of protein dispersion by infusing gas into it through a glass frit of specific porosity. The gas flow and duration of the bubbling are controlled. The foam is generated on the surface of the liquid and it rises in the glass tube where its height is followed in real time by image analysis using a charged-coupled device (CCD) camera. The amount of liquid incorporated into the foam and the drainage rate are followed by measuring the conductivity as a function of time and with reference to the conductivity of the solution before bubbling, in the cuvette (with the remaining liquid phase), and at electrodes placed at different heights of the tube (Kato A, Takahashi A, Matsudomi N, Kobayashi K. Determination of foaming properties of proteins by conductivity measurements. 1983. J Food Sci, 48, 62-65).

BLG-AITC conjugates were prepared as described in example 1 and then acidified to pH 7.0 or 4.0 by adding 1M HCl. The amount of HCl added was ~0.02 % and ~0.8 % of the total volume, for neutral and acidic pH adjustment, respectively. 12 mL of each sample at both pHs, was put into 12 mL-glass vial and heated on 85°C for 15 min, in addition to 5 min necessary for temperature in the vial to reach 85°C. The heating was done without agitation, in a temperature-controlled water-bath. Subsequently, samples were let to cool down at room temperature over 40 minutes under a mild agitation, and then put on ice until further analysis.

A volume of 20 mL of the sample solution (non-heated and heated BLG-AITC covalent complexes with the AITC to BLG molar ratio 0.5, 1 and 2, at both pH), diluted to 55 µM BLG (0.1%) was placed in the cuvette. Nitrogen at the flow rate 80 mL/min was bubbled in through the glass frit of porosity 4, creating the air bubbles with diameters between 10 and 16 µm. This flow rate allowed efficient foam formation before strong gravitational drainage occurred. The bubbling was stopped when the foam volume of 110 cm³ was reached. Subsequently, foam capacity (FC = volume of foam/volume of gas injected) and foam expansion (FE = volume of the foam/volume of the liquid in the foam) were calculated. Foam volume and liquid stability (time for the foam to drain 50% of its initial liquid content) were followed over next 30 min at 24±1°C.

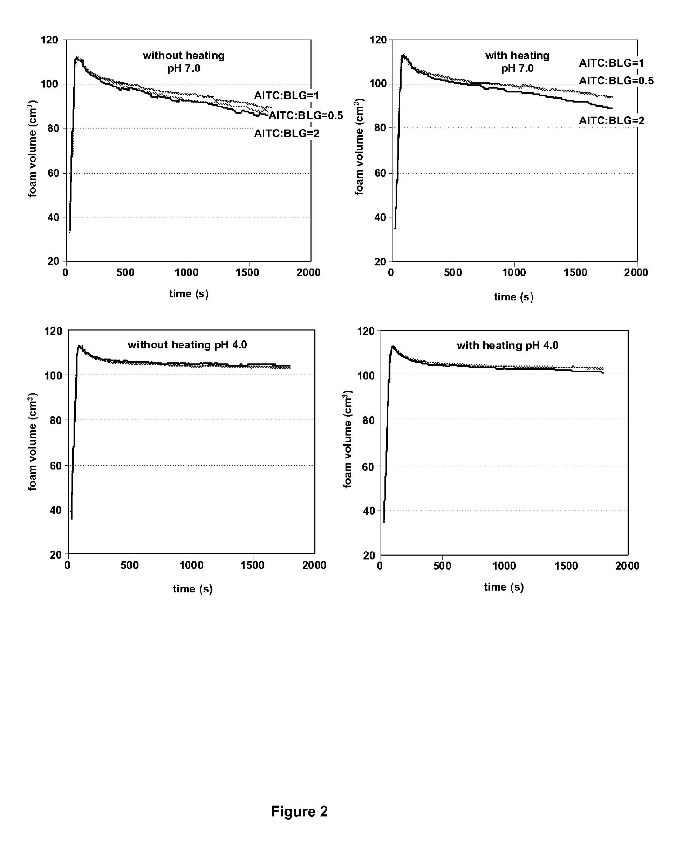

Figure 2 shows that volume stable foams could be obtained at pH 7.0 using BLG-AITC covalent complexes with or without heat treatment applied before foaming. This result was a clear indication of the air/water surface activity of the complexes. The foam volume stability was slightly affected by the initial AITC/BLG molar ratio, but a ratio of 1 gave the best foam stability. In acidic conditions (pH 4.0), foams were more volume stable than at pH 7.0, without or with heating applied before foaming. This showed that the covalent complexes were more able to entrap gas and liquid in the foam in acidic conditions than in neutral ones. There was no significant effect of initial AITC/BLG molar ratio on foam stability.

Table 1 shows the foam expansion, foam capacity and foam liquid stability determined at pH 4.0 and 7.0 for non-heated and heated (85°C, 15 min) BLG-AITC covalent complexes at 25°C for molar mixing ratio of 0.5, 1 and 2. Data presented are mean values with corresponding standard deviation.

| | | **foam expansion** | **foam capacity** | **foam liquid stability (s)** |
|---|---|---|---|---|
| | AITC/BLG ratio 0.5 | 6.8±0.1 | 1.16±0.01 | 152.0±3.3 |
| **pH 7.0 without heating** | AITC/BLG ratio 1 | 6.7±0.1 | 1.16±0.01 | 159.0±1.8 |
| | AITC/BLG ratio 2 | 6.8±0.1 | 1.17±0.01 | 145.5±3.9 |
| | AITC/BLG ratio 0.5 | 6.7±0.1 | 1.14±0.01 | 133.0±4.7 |
| **pH 7.0 with heating** | AITC/BLG ratio 1 | 6.8±0.1 | 1.14±0.02 | 130.0±4.2 |
| | AITC/BLG ratio 2 | 6.7±0.1 | 1.16±0.01 | 128.0±3.8 |
| | AITC/BLG ratio 0.5 | 7.1±0.0 | 1.18±0.01 | 204.5±8.5 |
| | | | | |
| **pH 4.0 without heating** | AITC/BLG ratio 1 | 7.1±0.1 | 1.18±0.01 | 217.5±1.0 |
| | AITC/BLG ratio 2 | 7.0±0.0 | 1.18±0.01 | 216.3±5.0 |
| | AITC/BLG ratio 0.5 | 7.1±0.2 | 1.16±0.00 | 200.0±6.9 |
| **pH 4.0 with heating** | AITC/BLG ratio 1 | 7.3±0.2 | 1.17±0.01 | 190.5±2.4 |
| | AITC/BLG ratio 2 | 7.1±0.2 | 1.16±0.01 | 193.0±2.7 |

Table 1 shows that AITC:BLG covalent complexes at pH 7.0 exhibited lower foam expansion values than at pH 4.0, with or without heat treatment. This indicates that slightly wetter foams were obtained at pH 7.0. Foam capacities were always higher than 1, indicating that the volume of foam comprised a significant amount of liquid (14 to 18%), together with the injected nitrogen. Higher foam capacities were obtained for the non-heated samples. The foam liquid stability was significantly improved (more than 30% more time stability) for foam produced in acidic conditions. Like for the foam capacity, higher values were obtained when the covalent complexes were not submitted to a subsequent heat treatment.

### Example 3: Determination of the emulsifying properties of β-lactoglobulin/allyl isothiocyanate covalent complexes

Emulsifying properties of BLG-AITC conjugates, non-heated and heated at pH 7.0 and 4.0 (prepared as described in examples 1 and 2), were evaluated by preparing emulsions containing 10% (w/w) of "high oleic" sunflower oil (Oleifico SABO, Manno, Switzerland). Samples containing BLG-AITC conjugates in the equimolar ratio were diluted to 0.25 mM BLG and mixed with oil in a pyrex tube (D 25 mm, V 35 mL) to a final weight of 20 g. They were pre-homogenized using an Ultra Turrax^{®} T25 equipped with a S25N-10G dispersing head (IKA-Werke, Staufen, Germany) rotating at 16,000 rpm for 1 minute. Homogenization of emulsions was completed using an ultrasonic processor (Hielscher UP400S, power 400 W, frequency 24 kHz) and a sonotrode (diameter 7 mm, approx. length 100 mm, titanium) at room temperature for 75 seconds and 75% amplitude of the ultrasound maximal power. This emulsification time was enough to disperse the oil in small droplets and to avoid excessive heating of the sample. The temperature inside the samples did not surpass 50°C.

Stability of emulsions to creaming and flocculation was followed over 33 days of storage at 4°C.

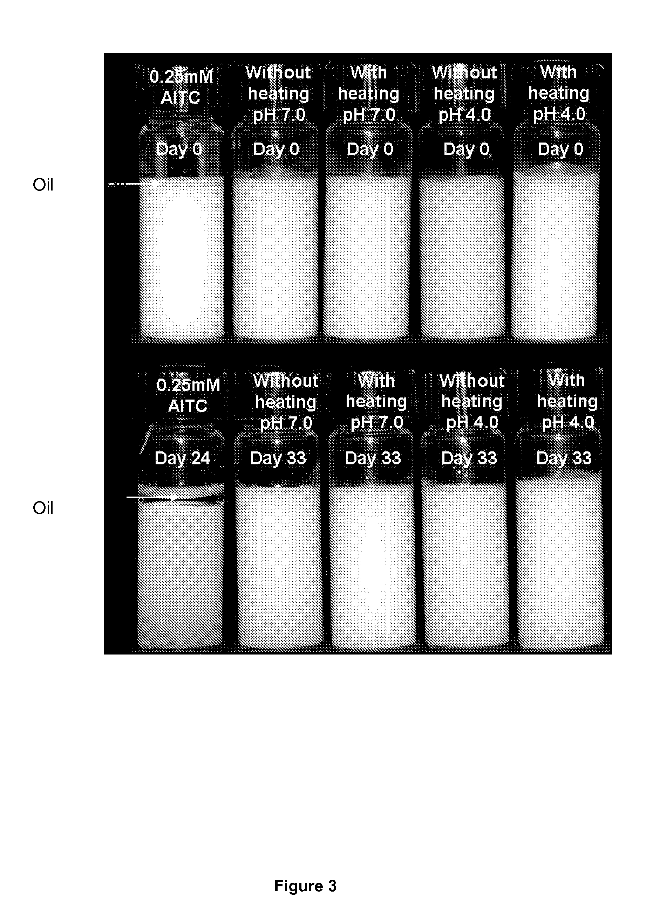

Figure 3 shows that AITC alone was not able to properly emulsify the 10% of sunflower oil used to produce the emulsion. Hence, significant oiling-off was visible already at day = 0 but more remarkably after 24 days of storage. Use of BLG-AITC covalent complexes led to very stable emulsions after 33 days of storage, either at neutral or acidic pH, with or without heat treatment applied. It can be concluded that BLG-AITC covalent complexes exhibit strong surface activity at the oil/water interface.

### Example 4: Determination of the pungency of β-lactoglobulin/allyl isothiocyanate covalent complexes

Eight subjects were instructed to comparatively score pungency, on the scale from 0 (not) to 10 (very), of solutions containing 0.5 mM AITC, 1 mM AITC, 0.5 mM BLG+0.5 mM AITC, and 0.5 mM BLG+1 mM AITC. Samples were presented in a randomized order in 2cl cups and coded. Subjects were instructed to first evaluate samples in a precise order (from left to right). Once they had tasted each sample, they were free to taste any sample another time. Vittel^{™} water at ambient temperature, bread or apple slices were available for mouth rinsing.

Samples for tasting were prepared as described in example 1, acidified to pH 7.0 and subsequently heated 15 min on 85°C. Pungency was first evaluated by sniffing and then in-mouth with a nose-clip. To determine if there was significant difference between samples, pair-wised t-tests was applied.

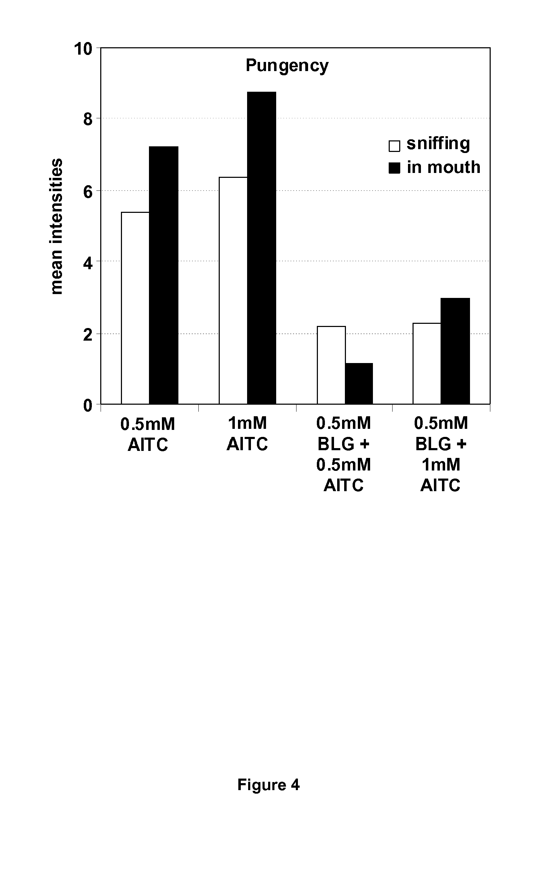

Figure 4 shows that formation of covalent complexes between AITC and BLG reduced significantly the pungency of the dispersions. This was shown by sniffing the samples and by in mouth tasting.

### Example 5: Determination of the anti-microbial activity of β-lactoglobulin/allyl isothiocyanate covalent complexes

Antimicrobial properties of BLG-AITC conjugates were tested against *Escherichia coli* and *Staphylococcus aureus.*

Single colony of each bacteria strain, previously grown on agar plates, was inoculated into 10 mL of brain-heart infusion (BHI) broth and incubated 24 h at 37°C under agitation. For antimicrobial properties evaluation these pre-cultures were diluted 100 times in BHI broth.

BLG-AITC covalent complexes, with the AITC to BLG molar ratio 1, 2, 10 and 20, were prepared as described in the example 1 with the difference that 200 mM AITC solution was prepared in DMSO. Samples were subsequently acidified to pH 7.0 and then concentrated 5 times by using Centrisart^{®} I centrifugal ultrafiltration units with cellulose triacetate ultrafilters having the molecular weight cut-off of 10 kDa (Sartorious, Germany) according to manufactures instructions.

An aliquot of 600 µL of concentrated samples was added to 2.4 mL of BHI broth containing E. *coli* or S. *aureus* to reach the starting concentrations of BLG (0.5mM) and corresponding concentration of AITC. Controls in which 600 µL of 5% DMSO or water was added were prepared as well. Test tubes with sample mixtures were then incubated 24 h at 37°C under stirring. Subsequently, optical densities (OD) of samples were measured at 600 nm. To evaluate the inhibition of bacteria growth, ODs of samples containing BLG-AITC covalent complexes were normalized by OD of the corresponding control and are expressed as the OD decrease in percentage. A solution of 1% DMSO did not inhibit growth of either of bacteria strains.

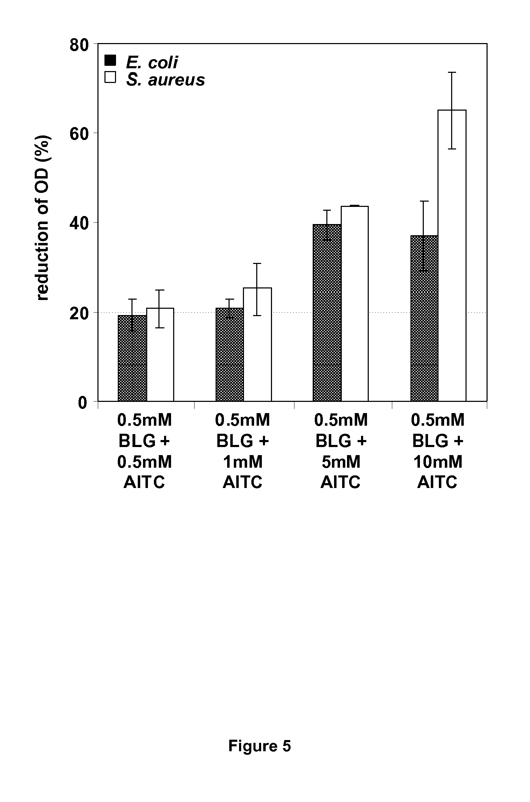

Figure 5 shows that BLG-AITC covalent complexes were able to reduce the optical density of the two strains of pathogens. This result can be interpreted to be a first sign for a reduction of the bacterial growth. It seemed that S. *aureus* was more sensitive to the covalent complexes than E. *coli.*

## Claims

1. Composition comprising a covalent complex of at least one isothiocyanate compound and at least one milk protein.

2. Composition in accordance with claim 1, wherein the milk protein is selected from the group consisting of the protein fraction of bovine milk, the protein fraction of skim milk, milk protein concentrate, milk protein isolate, micellar casein, caseinate, αₛ₁-casein, αₛ₂-casein, β-casein, κ-casein, the protein fraction of acid or sweet whey, whey protein concentrate, whey protein isolate, α-lactalbumin, β-lactoglobulin, bovine serum albumin and/or lactoferrin.

3. Composition in accordance with one of the preceding claims, wherein the isothiocyanate compounds is selected from the group consisting of organic isothiocyanates in the form of R-N=C=S, such as methyl isothiocyanate, allyl isothiocyanate, phenylethyl isothiocyanate, sulforaphane, phenethyl isothiocyanate, benzyl isothiocyanate, 3-methylthiopropyl isothiocyanate, and combinations thereof.

4. Composition in accordance with one of the preceding claims, wherein isothiocyanate and whey protein is present in the complex in a molar ratio in the range of about 100:1 to 1:10, preferably in a molar ratio in the range of about 10:1 to 1:10, most preferable in the range of about 1:1.

5. Composition in accordance with one of the preceding claims, wherein the composition has a pH in the range of about 2 to 9, preferably of about 3 to 7.

6. Composition in accordance with one of the preceding claims, wherein the composition comprises, preferably consists of, an emulsion, preferably an oil-in-water emulsion.

7. Composition in accordance with one of the preceding claims, wherein the composition comprises, preferably consists of, a foam.

8. Use of a composition in accordance with one of claims 1-7 to preserve isothiocyanates, in particular during storage times.

9. Use of a composition in accordance with one of claims 1-7 to produce an emulsion and/or a foam with a less pungent and/or lachrymatory taste from ITC-compounds.

10. Use of a composition in accordance with one of claims 1-7 to provide an antimicrobial effect, in particular to inhibit the growth of Escherichia coli and/or Staphylococcus aureus.

11. Use of a composition in accordance with one of claims 1-7 for the production of a product to treat or prevent disorders linked to microbial infection, in particular Escherichia coli and/or Staphylococcus aureus infection.

12. Use of a composition in accordance with one of claims 1-7 for the production of a product to inhibit carcinogenesis and/or tumorigenesis, in particular (UV) light-induced skin carcinogenesis, and/or to treat cystic fibrosis.

13. Use of a composition in accordance with one of claims 1-7 for the production of a product to treat or prevent inflammatory disorders, in particular inflammatory disorders than can be treated by phase 2 enzyme induction.

14. Use in accordance with one of claims 8-13, wherein the product is a food product, a neutraceutical, a food additive, a medicament, a cream for topical application or a drink.

15. Use in accordance with claim 14, wherein the food product is selected from the group consisting of a filling, dip, sauce (preferably mayonnaise), spread, topping, dairy-based product, a milk and/or cream based foam and/or emulsion, a salad dressing, a soup, a beverage or an oral food supplement.
